(19) Europäisches Patentamt
European Patent Office
Office européen des brevets

(11) Veröffentlichungsnummer: **0 272 444**
**A2**

(12) **EUROPÄISCHE PATENTANMELDUNG**

(21) Anmeldenummer: **87116856.3**

(22) Anmeldetag: **14.11.87**

(51) Int. Cl.⁴: **C07K 5/02** , A61K 37/64 , C07D 401/12 , A61K 31/395 , C07K 1/00 , //(C07D401/12, 213:00,207:00)

Die Bezeichnung der Erfindung wurde geändert (Richtlinien für die Prüfung im EPA, A-III, 7.3).

(30) Priorität: **27.11.86 DE 3640535**

(43) Veröffentlichungstag der Anmeldung: **29.06.88 Patentblatt 88/26**

(84) Benannte Vertragsstaaten: **AT BE CH DE ES FR GB IT LI NL SE**

(71) Anmelder: **Merck Patent Gesellschaft mit beschränkter Haftung**
**Frankfurter Strasse 250**
**D-6100 Darmstadt(DE)**

(72) Erfinder: **Hölzemann, Günter, Dr.**
**Weedring 5**
**D-6104 Seeheim 1(DE)**
Erfinder: **Raddatz, Peter, Dr.**
**Grafenstrasse 37**
**D-6100 Darmstadt(DE)**
Erfinder: **Schmitges, Claus J., Dr.**
**Karolinger Strasse 5**
**D-6114 Gross-Umstadt(DE)**
Erfinder: **Minck, Klaus-Otto, Dr.**
**Büchestrasse 8**
**D-6105 Ober-Ramstadt(DE)**
Erfinder: **Jonczyk, Alfred, Dr.**
**Jungfernstrasse 30**
**D-6100 Darmstadt 12(DE)**
Erfinder: **Sombroek, Johannes, Dr.**
**Robert-Koch-Strasse 32**
**D-6100 Darmstadt 13(DE)**
Erfinder: **Gante, Joachim, Dr.**
**Stormstrasse 4**
**D-6100 Darmstadt 12(DE)**

(54) **Renin inhibierende Peptide.**

(57) Neue Aminosäurederivate der Formel I

$$X\text{-}Z\text{-}NH\text{-}CHR^1\text{-}CO\text{-}NR^2\text{-}CHR^3\text{-}CR^4\text{-}(CHR^5)_n\text{-}CO\text{-}E\text{-}W'\text{-}Y \quad I$$

worin
X, Z, R¹, R², R³, R⁴, R⁵, E, W', Y und n die in Patentanspruch 1 angegebene Bedeutung haben, sowie ihre Slaze hemmen die Aktivität des menschlichen Plasmarenins.

EP 0 272 444 A2

# Peptide

Die Erfindung betrifft neue Aminosäurederivate der Formel I

$X-Z-NH-CHR^1-CO-NR^2-CHR^3-CR^4-(CHR^5)_n-CO-E-W'-Y$

worin

X H, $R-O-C_mH_{2m}-CO-$, $R-C_mH_{2m}-O-CO-$, $R-C_mH_{2m}-CO-$, $R-SO_2-$, $(R-C_mH_{2m})-L(R-C_pH_{2p})-C_rH_{2r}-CO-$, $R-(NHCH_2CH_2)_mNHCH_2CO-$oder 9-Fluorenyl-$C_mH_{2m}-O-CO-$,

Z 0 bis 4 peptidartig miteinander verbundene Aminosäurereste ausgewählt aus der Gruppe bestehend aus Abu, Ada, Ala, Arg, Asn, Asp, Bia, Cal, Dab, Gln, Glu, His, N(im)-Alkyl-His, Ile, Leu, tert.-Leu, Lys, Met, αNal, βNal, Nbg, Nle, Orn, Phe, Pro, Ser, Thr, Tic, Trp, Tyr und Val,

E 0 bis 2 peptidartig miteinander verbundene Aminosäurereste ausgewählt aus der Gruppe bestehend aus Abu, Ala, Cal, His, Ile, Leu, Met, Nle, Phe, Trp, Tyr und Val,

W' fehlt oder $-NH-CHR^6-CR^7-(CHR^8)_n-CO-$,

Y $-Q-C_tH_{2t}-R^9$ oder $NA_2$,

W'-Y auch

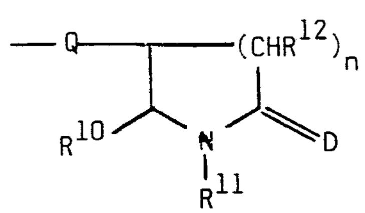

L CH oder N,

Q O oder $NR^{13}$,

D O oder S,

$R^1$ Pyridylmethyl oder Piperidylmethyl,

R, $R^3$, $R^6$, $R^9$ und $R^{10}$
jeweils H, A, Ar, Ar-alkyl, Het, Het-alkyl, unsubstituiertes oder ein-oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^2$, $R^5$, $R^8$, $R^{11}$, $R^{12}$ und $R^{13}$
jeweils H oder A,

$R^4$ und $R^7$
jeweils (H, OH), (H, $NH_2$) oder =O,

m, p, r und t
jeweils 0, 1, 2, 3, 4 oder 5,

n 1 oder 2,

Ar unsubstituiertes oder ein-oder mehrfach durch A, AO, Hal, $CF_3$, OH und/oder $NH_2$ substituiertes Phenyl, unsubstituiertes Naphthyl oder unsubstituiertes Pyridyl,

Het einen gesättigten oder ungesättigten 5-oder 6-gliedrigen heterocyclischen Rest mit 1-4 N-, O-und/oder S-Atomen, der mit einem Benzolring oder einem Pyridinring kondensiert und/oder ein-oder mehrfach durch A, AO, Hal, $CF_3$, HO, $O_2N$, Carbonylsauerstoff, $H_2N$, HAN, $A_2N$, AcNH, AS, ASO, $ASO_2$, AOOC, CN, $H_2NCO$, ANHCO, $A_2NCO$, ArNHCO, Ar-alkyl-NHCO, $H_2NSO_2$, $ASO_2NH$, Ar, Ar-alkyl, Ar-alkenyl, Hydroxyalkyl und/oder Aminoalkyl mit jeweils 1-8 C-Atomen substituiert sein kann und/oder dessen N-und/oder S-Heteroatome auch oxydiert sein können,

Hal F, Cl, Br oder J,

Ac H-CO-, A-CO-, Ar-CO-, A-NH-CO-oder Ar-NH-CO und

A Alkyl mit 1-8 C-Atomen bedeuten,

worin ferner an Stelle einer oder mehrerer -NH-CO-Gruppen auch eine oder mehrere -NA-CO-Gruppen stehen können,

worin jedoch die Gruppen E und W' nicht gleichzeitig fehlen dürfen,

sowie deren Salze.

Ähnliche Verbindungen sind aus der EP-A-152 255 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z. B. nach der Methode von F. Fyhrquist et. al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z. B. Pepsin und Kathepsin D) sind in der Regel wesentlich höhere Konzentrationen dieser Verbindungen notwendig.

Die Verbindungen können als Arzneimittelwirkstoffe in der Human-und Veterinärmedizin eingesetzt werden, insbesondere zur Prophylaxe und zur Behandlung von Herz-, Kreislauf-und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen.

Die vor-und nachstehend aufgeführten Abkürzungen von Aminosäureresten stehen für die Reste -NH-CHR-CO-(worin R die für jede Aminosäure bekannte spezifische Bedeutung hat) folgender Aminosäuren:

Abu 2-Aminobuttersäure
Ada Adamantylalanin
Ala Alanin
Arg Arginin
Asn Asparagin
Asp Asparaginsäure
Bia Benzimidazolylalanin
Cal Cyclohexylalanin
Dab 2,4-Diaminobuttersäure
Gln Glutamin
Glu Glutaminsäure
His Histidin
N(im)-Alkyl-His in 1-Stellung des Imidazolrings durch A substituiertes Histidin
Ile Isoleucin
Leu Leucin
tert.-Leu tert.-Leucin
Lys Lysin
Met Methionin
αNal α-Naphthylalanin
βNal β-Naphthylalanin
Nbg (2-Norbornyl)-glycin
Nle Norleucin
N-Me-His N-Methyl-histidin
N-Me-Phe N-Methyl-phenylalanin
Orn Ornithin
Phe Phenylalanin
Pia 3-(Piperidyl)-alanin, z.B. 2-Pia = 3-(2-Piperidyl)-alanin
Pro Prolin
Pya 3-(Pyridyl)-alanin, z.B. 3-Pya = 3-(3-Pyridyl)-alanin
Ser Serin
Thr Threonin
Tic Tetrahydroisochinolin-1-carbonsäure
Trp Tryptophan
Tyr Tyrosin
Val Valin.

Ferner bedeutet nachstehend:

BOC tert.-Butoxycarbonyl
imi-BOM Benzyloxymethyl in 1-Stellung des Imidazolrings
CBZ Benzyloxycarbonyl
DNP 2,4-Dinitrophenyl
imi-DNP 2,4-Dinitrophenyl in 1-Stellung des Imidazolrings
ETNC N-Ethylcarbamoyl
ETOC Ethoxycarbonyl
FMOC 9-Fluorenylmethoxycarbonyl
IPNC N-Isopropylcarbamoyl
IPOC Isopropoxycarbonyl
MC Morpholinocarbonyl
OMe Methylester
OEt Ethylester
PBB 4-Phenyl-2-benzylbutyryl
POA Phenoxyacetyl
DCCI Dicyclohexylcarbodiimid
HOBt 1-Hydroxybenzotriazol.

Sofern die vorstehend genannten Aminosäuren in mehreren enantiomeren Formen auftreten können, so sind vor-und nachstehend, z. B. als Bestandteil der Verbindungen der Formel I, alle diese Formen und auch ihre Gemische (z.B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Vor-und nachstehend haben die Reste bzw. Parameter X, Z, E, W', Y, L, Q, D, R, $R^1$ bis $R^{12}$, m, n, p, r, t, Ar, Het, Hal, Ac, A, $G^1$, $G^2$, $E^1$, $E^2$, $Z^1$, $Z^2$ und W die bei den Formeln I, II oder III angegebenen Bedeutungen, falls nicht ausdrücklich etwas anderes angegeben ist. Falls zwei Reste R in einer Verbindung der Formel I vorhanden sind, können sie gleich oder voneinander verschieden sein.

In den vorstehenden Formeln hat A 1 - 8, vorzugsweise 1,2,3 oder 4 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2-oder 3-Methylbutyl, 1,1-, 1,2-oder 2,2-Dimethylpropyl, 1-Ethylpropyl, Hexyl, 1-, 2-, 3-oder 4-Methylpentyl, 1,1-, 1,2-, 1,3-, 2,2-, 2,3-oder 3,3-Dimethylbutyl, 1-oder 2-Ethylbutyl, 1-Ethyl-1-methylpropyl, 1-Ethyl-2-methylpropyl, 1,1,2-oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z.B. 1-, 2-oder 3-Methylcyclopentyl, 1-, 2-, 3-oder 4-Methylcyclohexyl.

Dementsprechend bedeutet Cycloalkyl-alkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z.B. 1-, 2-oder 3-Methylcyclopentylmethyl, 1-, 2-, 3-oder 4-Methylcyclohexylmethyl.

Bicycloalkyl bedeutet vorzugsweise 1-oder 2-Dekalyl, 2-Bicyclo[2,2,1]heptyl oder 6,6-Dimethyl-2-bicyclo[3,1,1]heptyl.

Tricycloalkyl bedeutet vorzugsweise 2-Adamantyl.

Hal bedeutet vorzugsweise F, Cl oder Br, aber auch J.

Ac bedeutet vorzugsweise H-CO-, A-CO-wie Acetyl, Propionyl oder Butyryl, Ar-CO-wie Benzoyl, o-, m-oder p-Methoxybenzoyl oder 3,4-Dimethoxybenzoyl, A-NH-CO-wie N-Methyl-oder N-Ethylcarbamoyl, Ar-NH-CO-wie N-Phenylcarbamoyl.

Ar bedeutet vorzugsweise Phenyl, ferner bevorzugt o-, m-oder p-Tolyl, o-, m-oder p-Ethylphenyl, o-, m-oder p-Methoxyphenyl, o-, m-oder p-Fluorphenyl, o-, m-oder p-Chlorphenyl, o-, m-oder p-Bromphenyl, o-, m-oder p-Jodphenyl, o-, m-oder p-Trifluormethylphenyl, o-, m-oder p-Hydroxyphenyl, 2,3-, 2,4-, 2,5-, 2,6-, 3,4-oder 3,5-Dimethoxyphenyl, 3,4,5-Trimethoxyphenyl, o-, m-oder p-Aminophenyl, 1-oder 2-Naphthyl.

Dementsprechend bedeutet Ar-alkyl vorzugsweise Benzyl, 1-oder 2-Phenylethyl, o-, m-oder p-Methylbenzyl, 1-oder 2-o-, -m-oder -p-Tolylethyl, o-, m-oder p-Ethylbenzyl, 1-oder 2-o-, -m-oder -p-Ethylphenylethyl, o-, m-oder p-Methoxybenzyl, 1-oder 2-o-, -m-oder -p-Methoxyphenylethyl, o-, m-oder p-Fluorbenzyl, 1-oder 2-o-, -m-oder -p-Fluorphenylethyl, o-, m-oder p-Chlorbenzyl, 1-oder 2-o-, -m-oder -p-Chlorphenylethyl, o-, m-oder p-Brombenzyl, 1-oder 2-o-, -m-oder -p-Bromphenylethyl, o-, m-oder p-Jodbenzyl, 1-oder 2-o-, -m-oder -p-Jodphenylethyl, o-, m-oder p-Trifluormethylbenzyl, o-, m-oder p-Hydroxybenzyl, 2,3-, 2,4-, 2,5-, 2,6-, 3,4-oder 3,5-Dimethoxybenzyl, 3,4,5-Trimethoxybenzyl, o-, m-oder p-Aminobenzyl, 1-oder 2-Naphthylmethyl.

Het ist vorzugsweise 2-oder 3-Furyl, 2-oder 3-Thienyl, 1-, 2-oder 3-Pyrryl, 1-, 2-, 4-oder 5-Imidazolyl, 1-, 3-, 4-oder 5-Pyrazolyl, 2-, 4-oder 5-Oxazolyl, 3-, 4-oder 5-Isoxazolyl, 2-, 4-oder 5-Thiazolyl, 3-, 4-oder 5-Isothiazolyl, 2-, 3-oder 4-Pyridyl, 2-, 4-, 5-oder 6-Pyrimidyl, weiterhin bevorzugt 1,2,3-Triazol-1-, -4-oder -5-yl, 1,2,4-Triazol-1-, -3-oder -5-yl, 1-oder 5-Tetrazolyl, 1,2,3-Oxadiazol-4-oder -5-yl, 1,2,4-Oxadiazol-3-oder -5-yl, 1,3,4-Thiadiazol-2-oder -5-yl, 1,2,4-Thiadiazol-3-oder -5-yl, 2,1,5-Thiadiazol-3-oder -4-yl, 2-, 3-, 4-, 5-oder 6-2H-Thiopyranyl, 2-, 3-oder 4-4H-Thiopyranyl, 3-oder 4-Pyridazinyl, Pyrazinyl, 2-, 3-, 4-, 5-, 6 oder 7-Benzofuryl, 2-, 3-, 4-, 5-, 6-oder 7-Benzothienyl, 1-, 2-, 3-, 4-, 5-, 6-oder 7-Indolyl, 1-, 2-, 3-, 4-, 5-, 6-oder 7-Isoindolyl, 1-, 2-, 4-oder 5-Benzimidazolyl, 1-, 3-, 4-, 5-, 6-oder 7-Benzopyrazolyl, 2-, 4-, 5-, 6-oder 7-Benzoxazolyl, 3-, 4-, 5-, 6-oder 7-Benzisoxazolyl, 2-, 4-, 5-, 6-oder 7-Benzthiazolyl, 2-, 4-, 5-, 6-oder 7-Benzisothiazolyl, 4-, 5-, 6-oder 7-Benz-2,1,3-oxadiazolyl, 2-, 3-, 4-, 5-, 6-, 7-, oder 8-Chinolyl, 1-, 3-, 4-, 5-, 6-, 7-oder 8-Isochinolyl, 1-, 2-, 3-, 4-oder 9-Carbazolyl, 1-, 2-, 3-, 4-, 5-, 6-, 7-, 8-oder 9-Acridinyl, 3-, 4-, 5-, 6-, 7-oder 8-Chinnolyl, 2-, 4-, 5-, 6-, 7-oder 8-Chinazolyl. Die heterocyclischen Reste konnen auch teilweise oder vollständig hydriert sein. Het kann also z. B. auch bedeuten 2,3-Dihydro-2-, -3-, -4-oder -5-furyl, 2,5-Dihydro-2-, -3-, -4-oder 5-furyl, Tetrahydro-2-oder -3-furyl, Tetrahydro-2-oder -3-thienyl, 2,3-Dihydro-1-, -2-, -3-, -4-oder -5-pyrryl, 2,5-Dihydro-1-, -2-, -3-, -4-oder -5-pyrryl, 1-, 2-oder 3-Pyrrolidinyl, Tetrahydro-1-, -2-oder -4-imidazolyl, 2,3-Dihydro-1-, -2-, -3-, -4-oder 5-pyrazolyl, 2,5-Dihydro-1-, -2-, -3-, -4-oder 5-pyrazolyl, Tetrahydro-1-, -3-, oder -4-pyrazolyl, 1,4-Dihydro-1-, -2-, -3-oder -4-pyridyl, 1,2,3,4-Tetrahydro-1-, -2-, -3-, -4-, -5-oder -6-pyridyl, 1,2,3,6-Tetrahydro-1-, -2-, -3-, -4-, -5-oder -6-pyridyl, 1-, 2-, 3-oder 4-Piperidinyl, 2-, 3-oder 4-Morpholinyl, Tetrahydro-2-, -3-oder -4-pyranyl, 1,4-Dioxanyl, 1,3-Dioxan-2-, -4-oder -5-yl, Hexahydro-1-, -3-oder -4-pyridazinyl, Hexahydro-1-, -2-, -4-oder -5-pyrimidyl, 1-, 2-oder 3-Piperazinyl, 1,2,3,4-Tetrahydro-1-, -2-, -3-, -4-, -5-, -6-, -7-, oder -8-chinolyl, 1,2,3,4-Tetrahydro-1-, -2-, -3-, -4-, -5-, -6-, -7-oder 8-isochinolyl.

Die heterocyclischen Reste können auch wie angegeben substituiert sein. Het kann also bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 2-Amino-5,6-dimethyl-3-pyrazinyl, 4-Carbamoylpiperidino, ferner z.B. 3-, 4-oder 5-Methyl-2-furyl, 2-, 4-oder 5-Methyl-3-furyl, 2,4-Dimethyl-3-furyl, 5-Nitro-2-furyl, 5-Styryl-2-furyl, 3-, 4-oder 5-Methyl-2-thienyl, 2-, 4-oder 5-Methyl-3-thienyl, 3-Methyl-5-tert.-butyl-2-thienyl, 5-Chlor-2-thienyl, 5-Phenyl-2-oder -3-thienyl, 1-, 3-, 4-oder 5-Methyl-2-pyrryl, 1-Methyl-4-oder -5-nitro-2-pyrryl, 3,5-Dimethyl-4-ethyl-2-pyrryl, 4-Methyl-5-pyrazolyl, 4-oder 5-Methyl-2-thiazolyl, 2-oder 5-Methyl-4-thiazolyl, 2-oder 4-Methyl-5-thiazolyl, 2,4-Dimethyl-5-thiazolyl, 3-, 4-, 5-oder 6-Methyl-2-pyridyl, 2-, 4-, 5-oder 6-Methyl-3-pyridyl, 2-oder 3-Methyl-4-pyridyl, 3-, 4-, 5-oder 6-Chlor-2-pyridyl, 2-, 4-, 5-oder 6-Chlor-3-pyridyl, 2-oder 3-Chlor-4-pyridyl, 2,6-Dichlorpyridyl, 2-Hydroxy-3-, -4-, -5-oder -6-pyridyl (= 1H-2-Pyridon-3-, -4-, -5-oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4-pyridon-5-yl, 5-oder 6-Methyl-4-pyrimidyl, 2,6-Dihydroxy-4-pyrimidyl, 5-Chlor-2-methyl-4-pyrimidyl, 2-Methyl-4-amino-5-pyrimidyl, 3-Methyl-2-benzofuryl, 2-Ethyl-3-benzofuryl, 7-Methyl-2-benzothienyl, 1-, 2-, 4-, 5-, 6-oder 7-Methyl-3-indolyl, 1-Methyl-5-oder -6-benzimidazolyl, 1-Ethyl-5-oder -6-benzimidazolyl, 3-, 4-, 5-, 6-, 7-oder 8-Hydroxy-2-chinolyl.

R bedeutet vorzugsweise A, insbesondere Methyl, Ethyl, Propyl, Isopropyl, Butyl, Isobutyl oder tert.-Butyl, weiterhin vorzugsweise Cyclopropyl, Cyclopentyl, Cyclohexyl, Phenyl, Benzyl oder Morpholino.

R' bedeutet vorzugsweise 2-, 3-oder 4-Pyridylmethyl, ferner 1-, 2-, 3-oder 4-Piperidylmethyl. Dementsprechend bedeutet die Gruppe -NH-CHR'-CO-vorzugsweise 2-, 3-oder 4-Pya, ferner 1-, 2-, 3-oder 4-Pia.

$R^2$, $R^5$, $R^8$, $R''$, und $R'^3$ bedeuten vorzugsweise H oder Methyl, ferner Ethyl, Propyl, Isopropyl, Butyl oder Isobutyl.

$R^3$ und $R^6$ bedeuten vorzugsweise Cyclohexylmethyl, ferner bevorzugt A, insbesondere Methyl, Ethyl, Propyl, Isopropyl, Butyl, Isobutyl, sek.-Butyl, Pentyl, Isopentyl (3-Methylbutyl) oder 2-Methylbutyl, Phenyl, Benzyl, p-Chlorbenzyl, 2-Cyclohexylethyl, Bicyclo[2,2,1]heptyl-2-methyl oder 6,6-Dimethylbicyclo[3,1,1]heptyl-2-methyl.

$R^4$ und $R^7$ sind vorzugsweise (H, OH), ferner bevorzugt (H, $NH_2$).

$R^9$ ist vorzugsweise H oder Het, insbesondere Pyridyl, 2-Amino-5,6-dimethyl-3-pyrazinyl oder 2-Methyl-4-amino-5-pyrimidyl, ferner auch o-, m-oder p-Aminomethylphenyl.

$R^{10}$ ist vorzugsweise H, Methyl, Ethyl, Isobutyl oder sek.-Butyl, ferner bevorzugt Propyl, Butyl, Cyclohexylmethyl oder Benzyl.

L ist vorzugsweise CH. Q ist vorzugsweise O oder NH. D ist vorzugsweise O.

m, p, r und t sind vorzugsweise 0, 1 oder 2; n ist vorzugsweise 1.

X bedeutet vorzugsweise H, POA, Alkoxycarbonyl wie ETOC, IPOC oder BOC, CBZ, Alkanoyl wie Acetyl, Propionyl, Butyryl oder Isobutyryl, Cycloalkylcarbonyl wie Cyclopentylcarbonyl oder Cyclohexylcarbonyl, Aroyl wie Benzoyl, Arylalkanoyl wie Phenylacetyl, 2-oder 3-Phenylpropionyl, 4-Phenylbutyryl, 2-Benzyl-3-phenylpropionyl, PBB, 2-(2-Phenylethyl)-4-phenylbutyryl, 2-(2-Naphthylmethyl)-4-phenylbutyryl, 2-oder 3-o-, -m-oder -p-Fluorphenylpropionyl, 2-oder 3-o-, -m-oder -p-Chlorphenylpropionyl, Cycloalkylalkanoyl wie Cyclohexylacetyl, 2-oder 3-Cyclohexylpropionyl, N-Alkylcarbamoyl wie ETNC oder IPNC, MC. Besonders bevorzugte Reste X sind BOC und MC, weiterhin ETOC, IPOC, ETNC, IPNC und PBB, ferner H, POA, 4-Phenylbutyryl, 2-Benzyl-3-phenylpropionyl, 2-(2-Phenylethyl)-4-phenylbutyryl, 2-(2-Naphthylmethyl)-4-phenylbutyryl und CBZ.

Z bedeutet vorzugsweise 1, aber auch 0 oder 2, weiterhin 3 oder 4 peptidartig miteinander verbundene Aminosäurereste, insbesondere eine der Gruppen Phe, Pro-Phe oder His-Pro-Phe, ferner bevorzugt die Gruppen Abu, Ada, Arg, Asn, Bia, Cal, Dab, Gln, Glu, His, Ile, N-(im)-Methyl-His, Leu, tert.-Leu, Lys, Met, αNal, βNal, Nbg, Nle, Orn, N-Me-Phe, Pro, Ser, Thr, Tic, Trp, Tyr, Val, Ada-Phe, Ala-His, Ala-Phe, Phe-Abu, Phe-Ada, Phe-Ala, Phe-Arg, Phe-Asn, Phe-Bia, Phe-Cal, Phe-Dab, Phe-Gln, Phe-Glu, Phe-(N-im-Methyl-His), Phe-Ile, Phe-Leu, Phe-tert.-Leu, Phe-Lys, Phe-Met, Phe-α-Nal, Phe-βNal, Phe-Nbg, Phe-Nle, Phe-(N-Me-His), Phe-(N-Me-Phe), Phe-Orn, Phe-Phe, Phe-Pro, Phe-Ser, Phe-Thr, Phe-Tic, Phe-Trp, Phe-Tyr, Phe-Val, Pro-Ala, Pro-Ala-Phe, Pro-Phe-Ala, Pro-Phe-Phe, His-Pro-Ala, His-Pro-Ala-Phe, His-Pro-Phe-Ala, His-Pro-Phe-Phe, weiterhin Pro-Abu, Pro-Ada, Pro-Arg, Pro-Asn, Pro-Bia, Pro-Dab, Pro-Glu, Pro-His, Pro-Ile, Pro-Leu, Pro-tert.-Leu, Pro-Lys, Pro-Met, Pro-Nbg, Pro-Nle, Pro-(N-Me-His), Pro-(N-Me-Phe), Pro-Orn, Pro-Phe-Abu, Pro-Phe-Ada, Pro-Phe-Arg, Pro-Phe-Asn, Pro-Phe-Bia, Pro-Phe-Dab, Pro-Phe-Gln, Pro-Phe-Glu, Pro-Phe-(N-im-Methyl-His), Pro-Phe-Ile, Pro-Phe-Leu, Pro-Phe-tert.-Leu, Pro-Phe-Lys, Pro-Phe-Met, Pro-Phe-Nbg, Pro-Phe-Nle, Pro-Phe-(N-Me-His), Pro-Phe-(N-Me-Phe), Pro-Phe-Orn, Pro-Phe-Pro, Pro-Phe-Ser, Pro-Phe-Thr, Pro-Phe-Tic, Pro-Phe-Trp, Pro-Phe-Tyr, Pro-Phe-Val, Pro-Pro-His, Pro-Ser, Pro-Thr, Pro-Tic, Pro-Trp, Pro-Tyr, Pro-Val, His-Pro-Abu, His-Pro-Ada, His-Pro-Arg, His-Pro-Asn, His-Pro-Bia, His-Pro-Dab, His-Pro-Glu, His-Pro-His, His-Pro-Ile, His-Pro-Leu, His-Pro-tert.-Leu, His-Pro-Lys, His-Pro-Met, His-Pro-Nbg, His-Pro-Nle, His-Pro-(N-Me-His), His-Pro-(N-Me-Phe), His-Pro-Orn, His-Pro-Phe-Abu, His-Pro-Phe-Ada, His-Pro-Phe-Arg, His-Pro-Phe-Asn, His-Pro-Phe-Bia, His-Pro-Phe-Dab, His-Pro-Phe-Gln, His-Pro-Phe-Glu, His-Pro-Phe(N-im-Methyl-His), His-Pro-Phe-Ile, His-Pro-Phe-Leu, His-Pro-Phe-tert.-Leu, His-Pro-Phe-Lys, His-Pro-Phe-Met, His-Pro-Phe-Nbg, His-Pro-Phe-Nle, His-Pro-Phe-(N-Me-His), His-Pro-Phe-(N-Me-Phe), His-Pro-Phe-Orn, His-Pro-Phe-Pro, His-Pro-Phe-Ser, His-Pro-Phe-Thr, His-Pro-Phe-Tic, His-Pro-Phe-Trp, His-Pro-Phe-Tyr, His-Pro-Phe-Val, His-Pro-Pro, His-Pro-Ser,His-Pro-Thr, His-Pro-Tic, His-Pro-Trp, His-Pro-Tyr, His-Pro-Val.

E ist vorzugsweise abwesend oder bedeutet vorzugsweise Ile oder Leu, ferner bevorzugt Abu, Cal, Met oder Nle.

Die Gruppen W und W' bedeuten vorzugsweise $-NH-CHR^3-CHOH-CH_2-CO-$bzw. $-NH-CHR^6-CHOH-CH_2-CO-$, insbesondere $-NH-CH$(Cyclohexylmethyl)$-CHOH-CH_2-CO-$("AHCP", abgeleitet von 4-Amino-3-hydroxy-5-cyclohexylpentansäure), ferner $-NH-CH-(CH_2CH_2$-Cyclohexyl)$-CHOH-CH_2-CO-$("AHCH"; abgeleitet von 4-Amino-3-hydroxy-6-cyclohexylhexansäure), $-NH-CH$(Isobutyl)$-CHOH-CH_2-CO-$("Sta"; abgeleitet von Statin) oder $-NH-CH$(Benzyl)$-CHOH-CH_2-CO$ ("AHPP"; abgeleitet von 4-Amino-3-hydroxy-5-phenylpentansäure). Die Gruppen W und W' bedeuten ferner bevorzugt $-NH-CHR^3-CH(NH_2)-CH_2-CO-$bzw. $-NH-CHR^6-CH(NH_2)-CH_2-CO-$, insbesondere $-NH-CH$(Cyclohexylmethyl)$-CH(NH_2)-CH_2-CO-$("DACP"; abgeleitet von 3,4-Diamino-5-cyclohexylpentansäure), $-NH-CH(CH_2CH_2$-Cyclohexyl)$-CH(NH_2)-CH_2-CO-$("DACH"; abgeleitet von 3,4-Diamino-6-cyclohexylhexansäure), $-NH-CH$-(Isobutyl)$-CH(NH_2)-CH_2-CO-$("DAMH"; abgeleitet von 3,4-Diamino-6-methylheptansäure) oder $-NH-CH$-(Benzyl)$-CH(NH_2)-CH_2-CO-$("DAPP"; abgeleitet von 3,4-Diamino-5-phenylpentansäure).

Die Gruppen W und W' besitzen jeweils mindestens ein chirales Zentrum. Die Verbindungen der Formel I können daher in verschiedenen - optisch-inaktiven oder optisch-aktiven - Formen vorkommen. Die Formel I umschließt alle diese Formen.

Falls W $-NH-CHR^3-CR^4-CH_2-CO-$mit $R^4$ = (H, OH) oder (H, $NH_2$) bzw. falls W' $-NH-CHR^6-CR^7-CH_2-CO-$mit $R^7$ = (H, OH) oder (H, $NH_2$) bedeutet, sind die 3S-Hydroxy-4S-amino-Enantiomeren bzw. 3S,4S-Diamino-Enantiomeren bevorzugt. Wenn bei der Bezeichnung von Einzelsubstanzen nichts anderes angegeben ist, beziehen sich die Abkürzungen AHCP, AHCH, Sta, AHPP, DACP, DACH, DAMH und DAPP immer auf die 3S,4S-Formen.

Y ist vorzugsweise OH, OMe, OEt, $NH_2$, Pyridyl-methyl-amino (insbesondere 3-Pyridyl-methyl-amino), 4-Amino-2-methyl-5-pyrimidyl-methyl-amino oder 2-Amino-5,6-dimethyl-3-pyrazinyl-methyl-amino.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ic ausgedrückt werden, die der Formel I entsprechen, worin jedoch

in Ia
X H, ETOC, IPOC, BOC, CBZ, ETNC, IPNC oder MC,
Z Phe, Cal, αNal oder βNal,
$R^2$ und $R^5$ H,
$R^3$ Isobutyl, Cyclohexylmethyl, 2-Cyclohexylethyl oder Benzyl,
n 1,
E Ile, Leu oder Nle bedeuten und
W' fehlt oder AHCP, AHCH, Sta, AHPP, DACP, DACH, DAMH oder DAPP bedeutet;

in Ib
X H, ETOC, IPOC, BOC, ETNC, IPNC oder MC,
Z Phe, Cal, αNal oder βNal
$R^1$ Pya,
$R^2$ und $R^5$ H,
$R^3$ Cyclohexylmethyl,
$R^4$ (H, OH),
n 1,
E Ile, Leu oder Nle bedeuten und
W' fehlt oder DAMH bedeutet;

in Ic
X BOC,
Z Phe,
$R^1$ 3-Pya
$R^2$ und $R^5$ jeweils H,
$R^3$ Cyclohexylmethyl,
$R^4$ (H, OH),
n 1 und
E Ile bedeuten und W' fehlt.

Ferner sind bevorzugt Verbindungen der Formeln I' sowie Ia' bis Ic', die den Formeln I sowie Ia bis Ic entsprechen, worin jedoch
Y OH, OMe, OEt, $NH_2$, $NH-CH_2$-(3-pyridyl), $NH-CH_2$-(4-amino-2-methyl-5-pyrimidyl) oder $NH-CH_2$-(2-amino-5,6-dimethyl-3-pyrazinyl) bedeutet.

Gegenstand der Erfindung ist ferner ein Verfahren zur Herstellung eines Peptids der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Verbindung, die der Formel I entspricht, aber an Stelle von H-Atomen eine oder mehrere zusätzliche C-C-und/oder C-N-und/oder C-O-Bindungen und/oder O-Atome enthält, reduziert oder daß man eine Carbonsäure der Formel II

$X-G^1-OH$ II

worin $G^1$
(a) $-Z^1-$,
(b) -Z-,
(c) $-Z-NH-CHR^1-CO-$,
(d) $-Z-NH-CHR^1-CO-W-$,
(e) $-Z-NH-CHR^2-CO-W-E^1-$,
(f) $-Z-NH-CHR^1-CO-W-E-$
(g) $-Z-NH-CHR^1-CO-W-E-W'-$und W - $NR^2-CHR^3-CR^4-(CHR^5)_n-CO-$bedeuten
mit einer Aminoverbindung der Formel III

$H-G^2$ III

worin
$G^2$

(a) $-Z^2-NH-CHR^1-CO-W-E-W'-Y$,
(b) $-NH-CHR^1-CO-W-E-W'-Y$,
(c) -W-E-W'-Y,
(d) -E-W'-Y,
(e) $-E^2-W'-Y$,
(f) -W'-Y,
(g) -Y (worin Q = NH ist), $E^1$ + $E^2$ zusammen E und
$Z^1$ + $Z^2$ zusammen Z bedeuten
umsetzt,
und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder zur Herstellung einer Verbindung der Formel I, worin $R^4$ und/oder $R^7$ = (H, OH) oder (H, $NH_2$) bedeuten, ein Aminoketosäurederivat der Formel I, worin $R^4$ und/oder $R^7$ = O bedeuten, reduziert oder reduktiv aminiert und/oder einen Rest Y durch Behandeln mit veresternden, solvolysierenden oder amidierenden Mitteln in einen anderen Rest Y umwandelt und/oder eine Verbindung der Formel I durch Behandeln mit einer Säure in eines ihrer Salze überführt.

Die Verbindungen der Formel I und auch die Ausgangsstoffe zu ihrer Herstellung werden im

übrigen nach an sich bekannten Methoden hergestellt. wie sie in der Literatur (z. B. in den Standardwerken wie Houben-Weyl, Methoden der organischen Chemie, Georg-Thieme-Verlag, Stuttgart: ferner EP-A-45665, EP-A-77028, EP-A-77029, EP-A-81783, EP-A-152 255) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino-und oder Hydroxygruppen entsprechende geschützte Amino- und oder Hydroxygruppen enthalten, vorzugsweise solche, die an Stelle eines H-Atoms, das mit einem N-Atom verbunden ist, eine Aminoschutzgruppe tragen, z.B. solche, die der Formel I entsprechen, aber an Stelle einer His-Gruppe eine N(im)-$R'^4$-His-Gruppe (worin $R'^4$ eine Aminoschutzgruppe bedeutet, z.B. BOM oder DNP) enthalten, oder solche der Formel X-Z-CHR'-CO-$NR^2$-$CHR^3$-CH($NHR'^4$)-$(CHR^5)_n$-CO-E-W'-Y.

Ferner sind Ausgangsstoffe bevorzugt, die an Stelle des H-Atoms einer Hydroxygruppe eine Hydroxyschutzgruppe tragen, z. B. solche der Formel X-Z-NH-CHR'-CO-$NR^2$-$CHR^3$-$CHOR'^5$-$(CHR^5)_n$-CO-E-W'-Y, worin $R'^5$ eine Hydroxyschutzgruppe bedeutet.

Es können auch mehrere - gleiche oder verschiedene - geschützte Amino-und oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls duchgeführt worden ist. Typisch für solche Gruppen sind insbesondere unsubstituierte oder substituierte Acyl-, Aryl-(z.B. DNP), Aralkoxymethyl-(z.B. BOM) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aroma tischen oder heterocyclischen Carbonsäuren oder Sulfonsäuren abgeleitete Acylgruppen sowie insbesondere Alkoxycarbonyl-, Aryloxycarbonyl-und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-Jodethoxycarbonyl; Aralkyloxycarbonyl wie CBZ ("Carbobenzoxy"), 4-Methoxybenzyloxycarbonyl, FMOC. Bevorzugte Aminoschutzgruppen sind DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl-oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z. B. in den genannten Standardwerken und Patentanmeldungen beschrieben sind.

Das In-Freiheit-Setzen der Verbindungen der Formel I aus ihren funktionellen Derivaten gelingt - je nach der benutzten Schutzgruppe - z. B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole

wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet. Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z.B. bevorzugt mit 40 %iger Trifluoressigsäure in Methylenchlorid oder mit etwa 3 bis 5 n HCl in Dioxan bei 15-30° abgespalten werden, die FMOC-Gruppe mit einer etwa 5-bis 20%igen Lösung von Dimethylamin, Diethylamin oder Piperidin in DMF bei 15-30°. Eine Abspaltung der DNP-Gruppe gelingt z.B. auch mit einer etwa 3-bis 10%igen Lösung von 2-Mercaptoethanol in DMF/Wasser bei 15-30°.

Hydrogenolytisch entfernbare Schutzgruppen (z.B. BOM, CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-bis 10 %igem Pd-C in Methanol bei 20-30°.

Die Verbindungen der Formel I sind auch erhältlich durch Reduktion entsprechender Verbindungen, die an Stelle von H-Atomen eine oder mehrere zusätzliche C-C-und/oder C-N-und/oder C-O-Bindungen und/oder O-Atome enthalten.

So sind z.B. Aminoverbindungen der Formel I, die einen Substituenten Ar = Aminophenyl enthalten, durch Reduktion der entsprechenden Nitroverbindungen erhalten werden, z.B. durch katalytische Hydrierung unter den vorstehend für die Hydrogenolyse genannten Bedingungen.

Verbindungen der Formel I können auch durch direkte Peptidsynthese aus einer Carbonsäure-(Formel II) und einer Aminkomponente (Formel III) erhalten werden. Als Carbonsäurekomponenten eignen sich z.B. solche der Teilformeln X-Z-OH, X-Z-NH-CR'-COOH, X-Z-NH-CR'-CO-W-OH, X-Z-NH-CR'-CO-W-E-OH oder X-Z-NH-CR'-CO-W-E-W'-OH, als Aminkomponenten solche der Teilformeln $H_2N$-CR'-CO-W-E-W'-Y, H-W-E-W'-Y, H-E-W'-Y, H-W'-Y oder H-Y (worin Q = NH ist, also $H_2N$-$C_tH_{2t}$-$R^9$). Die Peptidbindung kann aber auch innerhalb der Gruppe Z bzw. E geknüpft werden; dabei wird eine Carbonsäure der Formel X-$Z^1$-OH bzw. X-Z-NH-CR'-CO-W-$E^1$-OH mit einer Aminoverbindung der Formel H-$Z^2$-NH-CR'-CO-W-E-W'-Y bzw. H-$E^2$-W'-Y umgesetzt, wobei $Z^1$ + $Z^2$ = Z bzw. $E^1$ + $E^2$ = E ist. Dabei arbeitet man zweckmäßig nach üblichen Methoden der Peptid-Synthese, wie sie z.B. in HoubenWeyl, 1.c., Band 15/II, Seiten 1 bis 806 (1974) beschrieben sind.

Die Reaktion gelingt vorzugsweise in Gegenwart eines Dehydratisierungsmittels, z. B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z.B. solche, in denen reaktive Gruppen intermediär durch Schutzgruppen blockiert sind. Die Aminosäurederivate III können z.B. in Form ihrer aktivierten Ester verwendet werden, die zweckmäßig in situ gebildet werden, z.B. durch Zusatz von HOBt oder N-Hydroxysuccinimid.

Die Ausgangsstoffe der Formeln II und III sind großenteils bekannt. Sofern sie nicht bekannt sind, können sie nach bekannten Methoden, z.B. den oben angegebenen Methoden der Peptidsynthese und der Abspaltung von Schutzgruppen, hergestellt werden.

Gewünschtenfalls kann in einer Verbindung der Formel I eine funktionell abgewandelte Amino-und/oder Hydroxygruppe durch Solvolyse oder Hydrogenolyse nach einer der oben beschriebenen Methoden in Freiheit gesetzt werden.

So kann insbesondere eine Verbindung der Formel I, worin X von H verschieden ist, in eine Verbindung der Formel I (X = H) umgewandelt werden, zweckmäßig durch Hydrogenolyse, falls X = CBZ ist, sonst durch selektive Solvolyse. Falls X = BOC ist, kann man z.B. mit HCl in Dioxan bei Raumtemperatur die BOC-Gruppe abspalten.

Weiterhin können z. B. Ketoverbindungen der Formel I ($R^4$ und/oder $R^7$ = O) zu Verbindungen der Formel I ($R^4$ und/oder $R^7$ = (H, OH)) reduziert werden, beispielsweise mit einem komplexen Metallhydrid wie $NaBH_4$, das nicht gleichzeitig die Peptid-Carbonylgruppen reduziert, in einem inerten Lösungsmittel wie Methanol bei Temperaturen zwischen etwa -10 und +30°.

Ketoverbindungen der Formel I ($R^4$ und/oder $R^7$ = O) können auch durch reduktive Aminierung in Verbindungen der Formel I ($R^4$ und/oder $R^7$ = H, $NH_2$) übergeführt werden. Man kann ein-oder mehrstufig reduktiv aminieren. So kann man z.B. die Ketoverbindung mit Ammoniumsalzen, z.B. Ammoniumacetat, und $NaCNBH_3$ behandeln, vorzugsweise in einem inerten Lösungsmittel, z.B. einem Alkohol wie Methanol bei Temperaturen zwischen etwa 0 und 50°, insbesondere zwischen 15 und 30°. Weiterhin ist es möglich, die Ketoverbindung zunächst mit Hydroxylamin in üblicher Weise in das Oxim zu überführen und dieses, z.B. durch katalytische Hydrierung an Raney-Nickel, zum Amin zu reduzieren.

Weiterhin ist es möglich, einen Rest Y durch Behandeln mit veresternden, solvolysierenden oder amidierenden Mitteln in einen anderen Rest Y umzuwandeln. So kann man eine Säure verestern, z. B. mit Hilfe eines Alkohols der Formel A-OH oder eines Diazoalkans, z. B. Diazomethan, oder man kann einen Ester zur entsprechenden Säure verseifen, z. B. mit wäßrig-dioxanischer Natronlauge bei Raumtemperatur. Ferner kann man z. B. einen Ester durch Behandeln mit Amioniak oder mit einem Amin der Formel A-$NH_2$ oder $A_2NH$ in das entsprechende Amid überführen.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein-oder mehrbasige Carbon-, Sulfon-oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-oder 3-Phenylpropionsäure, Citronensäure, Gluconsäure, Ascorbinsäure, Nicotinsäure, Isonicotinsäure, Methan-oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z. B. Pikrate, können zur Isolierung und oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger-oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human-oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und oder Hilfsstoffe wie Konservierungs-, Stabilisierungs-und/oder Netzmittel, Emulgatoren, Salze zur Beeinflussung des osmotischen Druckes, Puffersubstanzen, Farb-und oder Aromastoffe enthalten. Sie können, falls erwünscht, auch einen oder mehrere weitere Wirkstoffe enthalten, z B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-77028 beschriebenen Verbindungen verabreicht, vorzugsweise in Dosierungen zwischen etwa 100 mg und 30 g, insbesondere zwischen 500 mg und 5 g pro Dosierungseinheit. Die tägliche Dosierung liegt vorzugsweise zwischen etwa 2 und 600 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere

der jeweiligen Erkrankung, welcher die Therapie gilt. Die parenterale Applikation ist bevorzugt.

Vor-und nachstehend sind alle Temperaturen in °C angegeben. In den nachfolgenden Beispielen bedeutet "übliche Aufarbeitung": Man gibt, falls erforderlich, Wasser hinzu, neutralisiert, extrahiert mit Ether oder Dichlormethan, trennt ab, trocknet die organische Phase mit Natriumsulfat, filtriert, dampft ein und reinigt durch Chromatographie an Kieselgel und/oder Kristallisation.

Beispiel 1

Ein Gemisch von 1 g N-[3S-Hydroxy-4S-(N-tert.-butoxycarbonyl-L-phenylalanyl-L-(N-imi-2,4-dinitrophenylhistidyl)-L-(3-pyridyl)-alanyl-amino)-5-cylohexyl-pentanoyl]-isoleucin ["BOC-Phe-(imi-DNP-His)-(3-Pya)-AHCP-Ile-OH": erhältlich durch Reaktion von BOC-(3-Pya)-OH mit H-AHCP-Ile-OMe in Gegenwart von N-Methylmorpholin/HOBt/DCCI zu BOC-(3-Pya)-AHCP-Ile-OMe, Abspaltung der BOC-Gruppe, Reaktion mit BOC-Phe-(imi-DNP-His)-OH zu BOC-Phe-(imi-DNP-His)-(3-Pya)-AHCP-Ile-OMe und Verseifung], 2 g 2-Mercaptoethanol, 20 ml DMF und 20 ml Wasser wird unter Rühren bei 20° mit wässeriger $Na_2CO_3$-Lösung auf pH 8 eingestellt und 2 Std. bei 20° gerührt. Nach üblicher Aufarbeitung erhält man N-[3S-Hydroxy-4S-(N-tert.-butoxycarbonyl-L-phenylalanyl-L-histidyl-L-(3-pyridyl)-alanyl-amino)-5-cyclohexylpentanoyl]-isoleucin ("BOC-Phe-His-(3-Pya)-AHCP-Ile-OH").

Analog erhält man durch Spalung der entsprechenden (imi-DNP-His)-Derivate:

BOC-Phe-His-(2-Pya)-AHCP-Ile-OH
BOC-Phe-His-(4-Pya)-AHCP-Ile-OH
BOC-Phe-His-(2-Pia)-AHCP-Ile-OH
BOC-Phe-His-(3-Pia)-AHCP-Ile-OH
BOC-Phe-His-(4-Pia)-AHCP-Ile-OH.

Beispiel 2

Man löst 1 g BOC-Phe-(imi-BOM-His)-(3-Pya)-AHCP-Ile-OMe [erhältlich aus BOC-Phe-(imi-BOM-His)-OH und H-(3-Pya)-AHCP-Ile-OMe] in 10 ml aus Methanol, hydriert 3 Std. an 0,5 g 10%ig. Pd-C bei 20° und 1 bar, filtriert, dampft ein und erhält nach üblicher Aufarbeitung BOC-Phe-His-(3-Pya)-AHCP-Ile-OMe.

Beispiel 3

Man löst 1 g 3S-CBZ-amino-4S-[BOC-Phe-(3-Pya)-AHCP-Ile-amino]-6-methylheptansäure-methylester ["BOC-Phe-(3-Pya)-AHCP-Ile-(CBZ-DAMH)-OMe"; F. 210-214°; erhältlich durch Reaktion von BOC-Phe-(3-Pya)-AHCP-OH mit 3S-CBZ-amino-4S-(H-Ile-amino)-6-methylheptansäure-methylester-hydrochlorid] in 10 ml Ethanol, hydriert an 0,5 g 10%igem Pd-C bei 20° und 1 bar bis zum Stillstand, dampft ein und erhält nach chromatographischer Reinigung BOC-Phe-(3-Pya)-AHCP-Ile-DAMH-OMe; F. 203-205°.

Analog erhält man durch Spaltung der entsprechenden CBZ-Derivate:

BOC-Phe-(3-Pya)-AHCP-Ile-DACP-OMe
BOC-Phe-(3-Pya)-AHCP-Ile-DACH-OMe
BOC-Phe-(3-Pya)-AHCP-Ile-DAPP-OMe.

Beispiel 4

Analog Beispiel 3 hydrogenolysiert man 3S-Hydroxy-4S-[2-BOC-Phe-amino-3-(1-CBZ-3-piperidyl)-propionylamino]-5-cyclohexylpentanoyl-isoleucin-methylester ["BOC-Phe-(3-CBZ-Pia)-AHCP-Ile-OMe"; erhältlich durch Hydrierung von BOC-(3-Pya)-OH zu BOC-(3-Pia)-OH, Reaktion mit CBZ-Cl zu BOC-(3-CBZ-Pia)-OH, Reaktion mit H-AHCP-Ile-OMe zu BOC-(3-CBZ-Pia)-AHCP-Ile-OMe, Abspaltung der BOC-Gruppe zu H-(3-CBZ-Pia)-AHCP-Ile-OMe und Umsetzung mit BOC-Phe-OH] zu BOC-Phe-(3-Pia)-AHCP-Ile-OMe.

Analog erhält man:

BOC-Phe-(2-Pia)-AHCP-Ile-OMe
BOC-Phe-(3-Pia)-AHCP-Ile-OMe.

Beispiel 5

Analog Beispiel 3 hydrogenolysiert man 3S-(CBZ-amino)-4S-[BOC-Phe-(3-CBZ-Pia)-AHCP-Ile-amino]-6-methylheptansäuremethylester [erhältlich durch Verseifung von BOC-Phe-(3-CBZ-Pia)-AHCP-OMe zur freien Säure und Reaktion mit 3S-(CBZ-amino)-4S-Ile-amino-6-methylheptansäuremethylester] zu BOC-Phe-(3-Pia)-AHCP-Ile-DAMH-OMe.

Beispiel 6

Eine Lösung von 1 g BOC-Phe-(3-Pya)-AHCP-Ile-(N-2-p-nitrophenyl-ethyl-amid) [erhältlich aus BOC-Phe-(3-Pya)-AHCP-Ile-OH und p-Nitrobenzylamin] in 50 ml Methanol wird an 1 g

5%ig. Pd-C bei 20° und 1 bar bis zur Aufnahme der berechneten Wasserstoffmenge hydriert. Man filtriert, dampft ein und erhält BOC-Phe-(3-Pya)-AHCP-Ile-(N-2-p-aminophenyl-ethyl-amid).

Beispiel 7

Zu einer Lösung von 5,96 g N-[3S-Hydroxy-4S-(2S-amino-3-(3-pyridyl)-propionylamino)-5-cyclohexylpentanoyl]-isoleucin-(N-4-amino-2-methyl-5-pyrimidinyl-methyl-amid) ["H-(3-Pya)-AHCP-Ile-(N-4-amino-2-methyl-5-pyrimidinyl-methyl-amid)"; erhältlich durch Abspalung der BOC-Gruppe aus BOC-AHCP-Ile-(N-4-amino-2-methyl-5-pyrimidinyl-methyl-amid (F. ab 225°, Zers.) mit HCl Dioxan, Reaktion mit BOC-(3-Pya)-OH zu BOC-(3-Pya)-AHCP-Ile-(N-4-amino-2-methyl-5-pyrimidinyl-methyl-amid (F. 165-172°) und erneute Abspaltung der BOC-Gruppe] in 60 ml Dichlormethan gibt man 1,01 g N-Methylmorpholin. Unter Rühren versetzt man mit 2,65 g BOC-Phe-OH, 1,35 g HOBt und einer Lösung von 2,06 g DCCI in 50 ml Dichlormethan, rührt 14 Std. bei 2-6°, filtriert den ausgeschiedenen Dicyclohexylharnstoff ab und dampft das Filtrat ein. Nach üblicher Aufarbeitung erhält man BOC-Phe-(3-Pya)-AHCP-Ile-(N-4-amino-2-methyl-5-pyrimidinyl-methyl-amid), F. 185-193°.

Analog erhält man aus H-(3-Pya)-AHCP-Ile-(N-2-amino-5,6-dimethyl-3-pyrazinyl-methyl-amid) [erhältlich aus BOC-AHCP-Ile-(N-2-amino-5,6-dimethyl-3-pyrazinyl-methyl-amid (F. 175°) über BOC-(3-Pya)-AHCP-Ile-(N-2-amino-5,6-dimethyl-3-pyrazinyl-methyl-amid (F. 175-185°)] das BOC-Phe-(3-Pya)-AHCP-Ile-(N-2-amino-5,6-dimethyl-3-pyrazinyl-methyl-amid, F. 192-199°.

Beispiel 8

Analog Beispiel 7 erhält man aus BOC-Pro-OH und H-Phe-(3-Pya)-AHCP-Ile-OMe das BOC-Pro-Phe-(3-Pya)-AHCP-Ile-OMe.

Beispiel 9

Analog Beispiel 7 erhält man aus BOC-Phe-(3-Pya)-OH und H-AHCP-Ile-(N-4-amino-2-methyl-5-pyrimidinyl-methyl-amid) das BOC-Phe-(3-Pya)-AHCP-Ile-(N-4-amino-2-methyl-5-pyrimidinyl-methyl-amid), F. 185-193°.

Analog erhält man mit H-AHCP-Ile-OMe:

BOC-Phe-(2-Pya)-AHCP-Ile-OMe
BOC-Phe-(3-Pya)-AHCP-Ile-OMe
BOC-Phe-(4-Pya)-AHCP-Ile-OMe
BOC-Phe-(2-Pia)-AHCP-Ile-OMe
BOC-Phe-(3-Pia)-AHCP-Ile-OMe
BOC-Phe-(4-Pia)-AHCP-Ile-OMe
ETOC-Phe-(3-Pya)-AHCP-Ile-OMe
IPOC-Phe-(3-Pya)-AHCP-Ile-OMe
CBZ-Phe-(3-Pya)-AHCP-Ile-OMe
ETNC-Phe-(3-Pya)-AHCP-Ile-OMe
IPNC-Phe-(3-Pya)-AHCP-Ile-OMe
MC-Phe-(3-Pya)-AHCP-Ile-OMe
POA-Phe-(3-Pya)-AHCP-Ile-OMe
FMOC-Phe-(3-Pya)-AHCP-Ile-OMe.

Analog erhält man mit H-AHCP-Ile-OEt die entsprechenden Ethylester.

Beispiel 10

Analog Beispiel 7 erhält man aus BOC-Phe-(3-Pya)-AHCP-OH und 4S-(H-Ile-amino)-5S-isobutyl-pyrrolidon (herstellbar aus H-Ile-OH und 4S-Amino-5S-isobutyl-pyrrolidon) das 4S-[BOC-Phe-(3-Pya)-AHCP-Ile-amino]-5S-isobutyl-pyrrolidon.

Analog erhält man

BOC-Phe-(3-Pya)-AHCP-Ile-$NH_2$, F. 221-222°
BOC-Phe-(3-Pya)-AHCP-Ile-(N-3-pyridyl-methyl-amid), F. 164-174°
BOC-Phe-(3-Pya)-AHCP-Ile-(N-2-amino-5,6-dimethyl-3-pyrazinyl-methyl-amid), F. 192-199°
BOC-Phe-(3-Pya)-AHCP-Ile-(N-4-amino-2-methyl-5-pyrimidinyl-methyl-amid), F. 185-193°
BOC-Phe-(3-Pya)-AHCP-Ile-(N-2-hydroxy-4,6-dimethyl-3-pyridyl-methyl-amid)
BOC-Phe-(3-Pya)-AHCP-Ile-(N-5-tetrazolyl-methyl-amid)

IPOC-Phe-(3-Pya)-AHCP-Ile-$NH_2$
IPOC-Phe-(3-Pya)-AHCP-Ile-(N-3-pyridyl-methyl-amid)
IPOC-Phe-(3-Pya)-AHCP-Ile-(N-2-amino-5,6-dimethyl-3-pyrazinyl-methyl-amid)
IPOC-Phe-(3-Pya)-AHCP-Ile-(N-4-amino-2-methyl-5-pyrimidinyl-methyl-amid)
IPNC-Phe-(3-Pya)-AHCP-Ile-$NH_2$
IPNC-Phe-(3-Pya)-AHCP-Ile-(N-3-pyridyl-methyl-amid)
IPNC-Phe-(3-Pya)-AHCP-Ile-(N-2-amino-5,6-dimethyl-3-pyrazinyl-methyl-amid)
IPNC-Phe-(3-Pya)-AHCP-Ile-(N-4-amino-2-methyl-5-pyrimidinyl-methyl-amid)

MC-Phe-(3-Pya)-AHCP-Ile-$NH_2$
MC-Phe-(3-Pya)-AHCP-Ile-(N-3-pyridyl-methyl-amid)
MC-Phe-(3-Pya)-AHCP-Ile-(N-2-amino-5,6-dimethyl-3-pyrazinyl-methyl-amid)
MC-Phe-(3-Pya)-AHCP-Ile-(N-4-amino-2-methyl-5-pyrimidinyl-methyl-amid)

BOC-Cal-(3-Pya)-AHCP-Ile-$NH_2$
BOC-Cal-(3-Pya)-AHCP-Ile-(N-3-pyridyl-methyl-amid)
BOC-Cal-(3-Pya)-AHCP-Ile-(N-2-amino-5,6-dimethyl-3-pyrazinyl-methyl-amid)
BOC-Cal-(3-Pya)-AHCP-Ile-(N-4-amino-2-methyl-5-pyrimidinyl-methyl-amid)

BOC-αNal-(3-Pya)-AHCP-Ile-$NH_2$
BOC-αNal-(3-Pya)-AHCP-Ile-(N-3-pyridyl-methyl-amid)
BOC-αNal-(3-Pya)-AHCP-Ile-(N-2-amino-5,6-dimethyl-3-pyrazinyl-methyl-amid)
BOC-αNal-(3-Pya)-AHCP-Ile-(N-4-amino-2-methyl-5-pyrimidinyl-methyl-amid)

BOC-βNal-(3-Pya)-AHCP-Ile-$NH_2$
BOC-βNal-(3-Pya)-AHCP-Ile-(N-3-pyridyl-methyl-amid)
BOC-βNal-(3-Pya)-AHCP-Ile-(N-2-amino-5,6-dimethyl-3-pyrazinyl-methyl-amid)
BOC-βNal-(3-Pya)-AHCP-Ile-(N-4-amino-2-methyl-5-pyrimidinyl-methyl-amid)

BOC-Phe-(3-Pya)-AHCP-Leu-$NH_2$
BOC-Phe-(3-Pya)-AHCP-Leu-(N-3-pyridyl-methyl-amid)
BOC-Phe-(3-Pya)-AHCP-Leu-(N-2-amino-5,6-dimethyl-3-pyrazinyl-methyl-amid)
BOC-Phe-(3-Pya)-AHCP-Leu-(N-4-amino-2-methyl-5-pyrimidinyl-methyl-amid)

BOC-Phe-(3-Pya)-AHCP-Nle-$NH_2$
BOC-Phe-(3-Pya)-AHCP-Nle-(N-3-pyridyl-methyl-amid)
BOC-Phe-(3-Pya)-AHCP-Nle-(N-2-amino-5,6-dimethyl-3-pyrazinyl-methyl-amid)
BOC-Phe-(3-Pya)-AHCP-Nle-(N-4-amino-2-methyl-5-pyrimidinyl-methyl-amid)

BOC-Phe-(3-Pya)-AHCH-Ile-$NH_2$
BOC-Phe-(3-Pya)-AHCH-Ile-(N-3-pyridyl-methyl-amid)
BOC-Phe-(3-Pya)-AHCH-Ile-(N-2-amino-5,6-dimethyl-3-pyrazinyl-methyl-amid)
BOC-Phe-(3-Pya)-AHCH-Ile-(N-4-amino-2-methyl-5-pyrimidinyl-methyl-amid)

BOC-Phe-(3-Pya)-Sta-Ile-$NH_2$
BOC-Phe-(3-Pya)-Sta-Ile-(N-3-pyridyl-methyl-amid)
BOC-Phe-(3-Pya)-Sta-Ile-(N-2-amino-5,6-dimethyl-3-pyrazinyl-methyl-amid)
BOC-Phe-(3-Pya)-Sta-Ile-(N-4-amino-2-methyl-5-pyrimidinyl-methyl-amid)

BOC-Phe-(3-Pya)-AHPP-Ile-$NH_2$
BOC-Phe-(3-Pya)-AHPP-Ile-(N-3-pyridyl-methyl-amid)
BOC-Phe-(3-Pya)-AHPP-Ile-(N-2-amino-5,6-dimethyl-3-pyrazinyl-methyl-amid)
BOC-Phe-(3-Pya)-AHPP-Ile-(N-4-amino-2-methyl-5-pyrimidinyl-methyl-amid).

Beispiel 11

Analog Beispiel 7 erhält man aus BOC-Phe-(3-Pya)-AHCP-Ile-OH und H-Phe-OMe das BOC-Phe-(3-Pya)-AHCP-Ile-Phe-OMe.

Beispiel 12

Analog Beispiel 7 erhält man aus BOC-Phe-(3-Pya)-AHCP-Ile-OH und 4R-Amino-5S-isobutylpyrrolidon ds 4R-[BOC-Phe-(3-Pya)-AHCP-Ile-amino]-5S-isobutylpyrrolidon.

Mit 2-Thioxo-4S-amino-5S-isobutylpyrrolidon erhält man analog das 2-Thioxo-4S-[BOC-Phe-(3-Pya)-AHCP-Ile-amino]-5S-isobutylpyrrolidon.

Analog erhält man mit m-Bis-(aminomethyl)-benzol:

BOC-Phe-(3-Pya)-AHCP-Ile-(N-m-aminomethyl-benzyl-amid)
IPOC-Phe-(3-Pya)-AHCP-Ile-(N-m-aminomethyl-benzyl-amid)
IPNC-Phe-(3-Pya)-AHCP-Ile-(N-m-aminomethyl-benzyl-amid)
MC-Phe-(3-Pya)-AHCP-Ile-(N-m-aminomethyl-benzyl-amid)
BOC-Cal-Phe-(3-Pya)-AHCP-Ile-(N-m-aminomethyl-benzyl-amid)
BOC-αNal-Phe-(3-Pya)-AHCP-Ile-(N-m-aminomethyl-benzyl-amid)
BOC-βNal-Phe-(3-Pya)-AHCP-Ile-(N-m-aminomethyl-benzyl-amid)
BOC-Phe-(3-Pya)-AHCP-Leu-(N-m-aminomethyl-benzyl-amid)
BOC-Phe-(3-Pya)-AHCP-Nle-(N-m-aminomethyl-benzyl-amid).

Beispiel 13

Analog Beispiel 7 erhält man aus BOC-Phe-(3-Pya)-AHCP-Ile-Sta-OH und 2-Amino-3-aminomethyl-5,6-dimethyl-pyrazin das BOC-Phe-(3-Pya)-AHCP-Ile-Sta-(N-2-amino-5,6-dimethyl-3-pyrazinyl-methyl-amid).

Beispiel 14

Eine Lösung von 1 g BOC-Phe-(3-Pya)-AHCP-Ile-OH in 20 ml 4 n HCl in Dioxan wird 30 Min. bei 20° gerührt und dann eingedampft. Man erhält H-Phe-(3-Pya)-AHCP-Ile-OH.

Analog erhält man durch Spaltung der entsprechenden N-BOC-Derivate:

H-Phe-(3-Pya)-AHCP-Ile-DAMH-OMe
H-Phe-(3-Pya)-AHCP-Ile-(N-3-pyridyl-methyl-amid)
H-Phe-(3-Pya)-AHCP-Ile-(N-4-amino-2-methyl-5-pyrimidinyl-methyl-amid)
H-Phe-(3-Pya)-AHCP-Ile-(N-2-amino-5,6-dimethyl-3-pyrazinyl-methyl-amid).

Beispiel 15

Man löst 1 g CBZ-Phe-(3-Pya)-AHCP-Ile-OH in 10 ml Ethanol, hydriert 3 Std. an 0.5 g 10%ig. Pd-C bei 20° und 1 bar, filtriert, dampft ein und erhält nach chromatographischer Reinigung H-Phe-(3-Pya)-AHCP-Ile-OH.

Beispiel 16

Eine Lösung von 1 g N-[3-Oxo-4S-(BOC-Phe-(3-Pya)-amino)-5-cyclohexyl-pentanoyl]-Ile-OH wird an 0.1 g 10%ig. Pd-C bei 20° und 1 bar bis zur Aufnahme der berechneten Menge Wasserstoff hydriert. Nach Filtrieren und Eindampfen erhält man ein Gemisch aus N-[3R-und N-[3S-Hydroxy-4S-(BOC-Phe-(3-Pya)-amino)-5-cyclohexyl-pentanoyl]-Ile-OH ["BOC-Phe-(3-Pya)-AHCP-Ile-OH"], das chromatographisch getrennt werden kann.

Beispiel 17

Eine Lösung von 721 mg N-[3-Oxo-4S-(2S-(N-tert.-butoxycarbonyl-L-phenylalanyl-amino)-3-(3-pyridyl)-propionyl-amino)-5-cyclohexylpentanoyl]-isoleucin und 1,43 g $Na_2CO_3$ . 10 $H_2O$ in 5 ml Methanol und 5 ml Wasser wird mit 70 mg Hydroxylaminhydrochlorid versetzt und 14 Std. bei 20° gerührt. Das ausgefallene Oxim wird abfiltriert, getrocknet, in 10 ml Methanol gelöst und an 0,5 g Raney-Ni bei 20° und 5 bar hydriert. Man filtriert den Katalysator ab, dampft das Filtrat ein, trennt an Kieselgel und erhält N-[3S-Amino-4S-(2S-(N-tert.-butoxycarbonyl-L-phenylalanyl-amino)-3-(3-pyridyl)-propionylamino)-5-cyclohexylpentanoyl]-isoleucin ["BOC-Phe-(3-Pya)-DACP-Ile-OH"]; daneben erhält man das 3R-Amino-Epimere.

Beispiel 18

Man rührt eine Lösung von 1 g BOC-Phe-(3-Pya)-AHCP-Ile-OMe in 50 ml Dioxan und 5 ml 2 n wässeriger Natronlauge 5 Std. bei 20°, dampft ein, nimmt mit Wasser auf, neutralisiert mit 1 n Salzsäure, arbeitet wie üblich auf (Ethylacetat) und erhält BOC-Phe-(3-Pya)-AHCP-Ile-OH.

Beispiel 19

Analog Beispiel 7 erhält man aus BOC-Pro-OH und H-Phe-(3-Pya)-AHCP-Ile-$NH_2$ das BOC-Pro-Phe-(3-Pya)-AHCP-Ile-$NH_2$, F. 158-162°.

Analog erhält man:

CBZ-Arg-Pro-Phe-(3-Pya)-AHCP-Ile-$NH_2$, F. 128°
[2R-Benzyl-4-(2-oxopyrrolidino)-butyryl]-(3-Pya)-AHCP-Ile-$NH_2$, F. 90-102°
[2S-Benzyl-4-(2-oxopyrrolidino)-butyryl]-(3-Pya)-AHCP-Ile-$NH_2$, F. 80-95°
MC-Phe-(3-Pia)-AHCP-Ile-(N-2-amino-5,6-dimethyl-3-pyrazinyl-methyl-amid), F. 146-150°.

Die nachstehenden Beispiele betreffen pharmazeutische Zubereitungen.

Beispiel A: Injektionsgläser

Eine Lösung von 100 g BOC-Phe-(3-Pya)-AHCP-Ile-(N-amino-2-methyl-5-pyrimidinyl-methylamid) und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 500 mg Wirkstoff.

Beispiel B: Suppositorien

Man schmilzt ein Gemisch von 500 g BOC-Phe-(3-Pya)-AHCP-Ile-DAMH-OMe mit 100 g Sojalecithin und 1400 g Kakaobutter, gießt in Formen und läßt erkalten. Jedes Suppositorium enthält 500 mg Wirkstoff.

## Ansprüche

1. Peptide der Formel I

X-Z-NH-CHR[1]-CO-NR[2]-CHR[3]-CR[4]-(CHR[5])$_n$-CO-E-W'-Y

worin

X   H, R-O-$C_mH_{2m}$-CO-, R-$C_mH_{2m}$-O-CO-,

$R-C_mH_{2m}-CO-$, $R-SO_2-$,
$(R-C_mH_{2m})-L(R-C_pH_{2p})-C_rH_{2r}-CO-$,
$R-(NHCH_2CH_2)_mNHCH_2CO-$oder
9-Fluorenyl-$C_mH_{2m}$-O-CO-,

Z 0 bis 4 peptidartig miteinander verbundene Aminosäurereste ausgewählt aus der Gruppe bestehend aus Abu, Ada, Ala, Arg, Asn, Asp, Bia, Cal, Dab, Gln, Glu, His, N(im)-Alkyl-His, Ile, Leu, tert.-Leu, Lys, Met, αNal, βNal, Nbg, Nle, Orn, Phe, Pro, Ser, Thr, Tic, Trp, Tyr und Val,

E 0 bis 2 peptidartig miteinander verbundene Aminosäurereste ausgewählt aus der Gruppe bestehend aus Abu, Ala, Cal, His, Ile, Leu, Met, Nle, Phe, Trp, Tyr und Val,

W' fehlt oder $-NH-CHR^6-CR^7-(CHR^8)_n-CO-$,

Y $-Q-C_tH_{2t}-R^9$ oder $NA_2$,

W'-Y auch

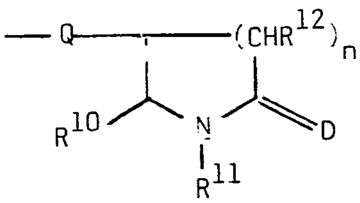

L CH oder N,

Q O oder $NR^{13}$,

D O oder S,

$R^1$ Pyridylmethyl oder Piperidylmethyl,

R, $R^3$, $R^6$, $R^9$ und $R^{10}$
jeweils H, A, Ar, Ar-alkyl, Het, Het-alkyl, unsubstituiertes oder ein-oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^2$, $R^5$, $R^8$, $R^{11}$, $R^{12}$ und $R^{13}$
jeweils H oder A,

$R^4$ und $R^7$
jeweils (H, OH), (H, $NH_2$) oder =O,

m, p, r und t
jeweils 0, 1, 2, 3, 4 oder 5,

n 1 oder 2,

Ar unsubstituiertes oder ein-oder mehrfach durch A, AO, Hal, $CF_3$, OH und/oder $NH_2$ substituiertes Phenyl, unsubstituiertes Naphthyl oder unsubstituiertes Pyridyl,

Het einen gesättigten oder ungesättigten 5- oder 6-gliedrigen heterocyclischen Rest mit 1-4 N-, O-und/oder S-Atomen, der mit einem Benzolring oder einem Pyridinring kondensiert und/oder ein- oder mehrfach durch A, AO, Hal, $CF_3$, HO, $O_2N$, Carbonylsauerstoff, $H_2N$, HAN, $A_2N$, AcNH, AS, ASO, $ASO_2$, AOOC, CN, $H_2NCO$, ANHCO, $A_2NCO$, ArNHCO, Ar-alkyl-NHCO, $H_2NSO_2$, $ASO_2NH$, Ar, Ar-alkyl, Ar-alkenyl, Hydroxyalkyl und/oder Aminoalkyl mit jeweils 1-8 C-Atomen substituiert sein kann und/oder dessen N-und/oder S-Heteroatome auch oxydiert sein können,

Hal F, Cl, Br oder J,

Ac H-CO-, A-CO-, Ar-CO-, A-NH-CO-oder Ar-NH-CO-und

A Alkyl mit 1-8 C-Atomen bedeuten,

worin ferner an Stelle einer oder mehrerer -NH-CO-Gruppen auch eine oder mehrere -NA-CO-Gruppen stehen können,
worin jedoch die Gruppen E und W' nicht gleichzeitig fehlen dürfen,
sowie deren Salze.

2. 3S-Amino-4S-[4S-(BOC-Phe-(L-(3-pyridyl)-alanyl)-amino)-3S-hydroxy-5-cyclohexyl-pentanoylamino-Ile-amino]-6-methylheptansäuremethylester.

3. Verfahren zur Herstellung eine Peptids der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt
oder daß man eine Verbindung, die der Formel I entspricht, aber an Stelle von H-Atomen eine oder mehrere zusätzliche C-C-und/oder C-N-und/oder C-O-Bindungen und/oder O-Atome enthält, reduziert
oder daß man eine Carbonsäure der Formel II

$X-G^1-OH$ II

worin $G^1$
(a) $-Z^1-$,
(b) -Z-,
(c) $-Z-NH-CHR^1-CO-$,
(d) $-Z-NH-CHR^1-CO-W-$,
(e) $-Z-NH-CHR^1-CO-W-E^1-$,
(f) $-Z-NH-CHR^1-CO-W-E-$
(g) $-Z-NH-CHR^1-CO-W-E-W'-$und

W $-NR^2-CHR^3-CR^4-(CHR^5)_n-CO-$bedeuten

mit einer Aminoverbindung der Formel III

$H-G^2$ III

worin
$G^2$
(a) $-Z^2-NH-CHR^1-CO-W-E-W'-Y$,
(b) $-NH-CHR^1-CO-W-E-W'-Y$,
(c) -W-E-W'-Y,
(d) -E-W'-Y,
(e) $-E^2-W'-Y$,
(f) -W'-Y,
(g) -Y (worin Q = NH ist),

$E^1$ + $E^2$ zusammen E und
$Z^1$ + $Z^2$ zusammen Z bedeuten

umsetzt,
und daß man gegebenenfalls in einer Verbindung der Formel I eine funktionell abgewandelte Amino-

und oder Hydroxygruppe durch Behandeln mit solvolysierenden oder hydrogenolysierenden Mitteln in Freiheit setzt und oder zur Herstellung einer Verbindung der Formel I, worin $R^4$ und oder $R^7$ = (H, OH) oder (H, $NH_2$) bedeuten, ein Aminoketosäurederivat der Formel I, worin $R^4$ und oder $R^7$ = O bedeuten, reduziert oder reduktiv aminiert und oder einen Rest Y durch Behandeln mit veresternden, solvolysierenden oder amidierenden Mitteln in einen anderen Rest Y umwandelt und oder eine Verbindung der Formel I durch Behandeln mit einer Säure in eines ihrer Salze überführt.

4. Verfahren zur Herstellung pharmazeutischer Zubereitungen, dadurch gekennzeichnet, daß man eine Verbindung der Formel I und oder eines ihres physiologisch unbedenklichen Salze zusammen mit mindestens einem festen, flüssigen oder halbflüssigen Träger-oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und oder einem ihrer physiologisch unbedenklichen Salze.

6. Verwendung von Verbindungen der Formel I oder von deren physiologisch unbedenklichen Salzen bei der Bekämpfung der reninabhängigen Hypertension oder des Hyperaldosteronismus.

7. Verwendung von Verbindungen der Formel I oder von deren physiologisch unbedenklichen Salzen zur Herstellung eines Arzneimittels.